(19) Europäisches Patentamt
European Patent Office
Office européen des brevets

(11) Veröffentlichungsnummer: **0 136 455**
**A1**

(12) # EUROPÄISCHE PATENTANMELDUNG

(21) Anmeldenummer: **84109024.4**

(22) Anmeldetag: **31.07.84**

(51) Int. Cl.⁴: **C 07 D 239/42**
C 07 D 239/46, A 01 N 47/44
//C07C143/78

(30) Priorität: **10.08.83 JP 145157/83**

(43) Veröffentlichungstag der Anmeldung: **10.04.85 Patentblatt 85/15**

(84) Benannte Vertragsstaaten: **AT BE CH DE FR GB IT LI NL SE**

(71) Anmelder: **NIHON TOKUSHU NOYAKU SEIZO K.K.**
**No.4, 2-chome, Nihonbashi Honcho Chuo-ku**
**Tokyo 103(JP)**

(72) Erfinder: **Shiokawa, Kozo**
**210-6, Shukugawara Tama-ku**
**Kawasaki-shi Kanagawa-ken(JP)**

(72) Erfinder: **Moriya, Koichi**
**39-15, Namiki-cho**
**Hachioji-shi Tokyo(JP)**

(72) Erfinder: **Goto, Toshio**
**20-1-304, 5-Chome, Seishin**
**Sagamihara-shi Kanagawa-ken(JP)**

(72) Erfinder: **Kamochi, Atsumi**
**2-24-10, Higashi-Toyoda**
**Hino-shi Tokyo(JP)**

(72) Erfinder: **Kohama, Shigeo**
**39-15, Namiki-cho**
**Hachioji-shi Tokyo(JP)**

(74) Vertreter: **Schumacher, Günter, Dr. et al,**
**c/o Bayer AG Konzernverwaltung RP Patentabteilung**
**D-5090 Leverkusen 1 Bayerwerk(DE)**

(54) **Substituierte Phenylsulfonylguanidin-Derivate.**

(57) Die Erfindung betrifft neue substituierte Phenylsulfonylguanidin-Derivate der allgemeinen Formel (I)

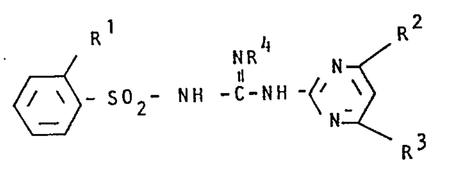

(I)

in der

$R^1$ für Phenyl oder Phenoxy steht,

$R^2$ und $R^3$ jeweils für niederes Alkyl oder niederes Alkoxy stehen und

$R^4$ für Wasserstoff, niederes Alkyl oder niederes Alkoxy steht,

ein Verfahren und neue Zwischenprodukte zu ihrer Herstellung und ihre Verwendung als Herbizide.

EP 0 136 455 A1

Nihon Tokushu Noyaku Seizo K.K., Tokyo/Japan 0136455

Bi-Ma
Ia

Substituierte Phenylsulfonylguanidin-Derivate

Die vorliegende Erfindung betrifft neue substituierte Phenylsulfonylguanidin-Derivate, Vorprodukte derselben, Verfahren zu ihrer Herstellung und Unkrautbekämpfungsmittel.

Insbesondere betrifft die vorliegende Erfindung substituierte Phenylsulfonylguanidin-Derivate, die durch die nachstehende Formel (I)

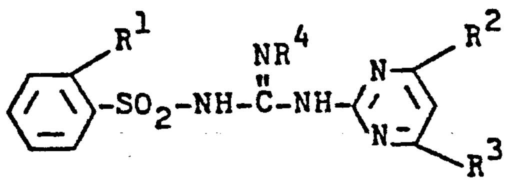
(I)

dargestellt werden, in der
$R^1$ eine Phenyl- oder Phenoxy-Gruppe bezeichnet,
$R^2$ und $R^3$ jeweils eine niedere Alkyl-Gruppe oder eine niedere Alkoxy-Gruppe bezeichnen und
$R^4$ ein Wasserstoff-Atom, eine niedere Alkyl-Gruppe oder eine niedere Alkoxy-Gruppe bezeichnet.

Die durch die allgemeine Formel (I) dargestellten Verbindungen gemäß der vorliegenden Erfindung können beispielsweise durch das nachstehende Verfahren (i) hergestellt werden, auf das sich die vorliegende Erfindung ebenfalls erstreckt:

Nit 163

Verfahren (i):

Das Verfahren (i) zur Herstellung der substituierten Phenylsulfonylguanidin-Derivate der allgemeinen Formel (I) umfaßt die Umsetzung einer durch die allgemeine Formel (II)

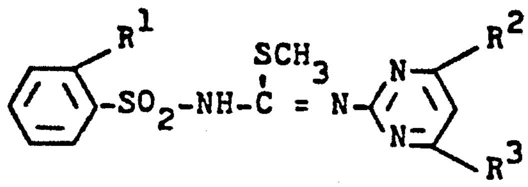

(II)

,

in der $R^1$, $R^2$ und $R^3$ die im Vorstehenden angegebenen Bedeutungen haben, bezeichneten Verbindung mit einer Verbindung der allgemeinen Formel

$R^4-NH_2$ (III),

in der $R^4$ die im Vorstehenden angegebene Bedeutung hat.

Die vorliegende Erfindung betrifft auch substituierte Phenylsulfonylisothioharnstoff-Derivate der allgemeinen Formel (II), die Vorprodukte des vorstehenden Verfahrens (i) sind, sowie die folgenden Verfahren (ii) und (iii) zur Herstellung der Verbindungen (II):

Verfahren (ii):

Das Verfahren (ii) zur Herstellung der substituierten Phenylsulfonylisothioharnstoff-Derivate der allgemeinen Formel (II) umfaßt die Umsetzung einer Verbindung der allgemeinen Formel

0136455

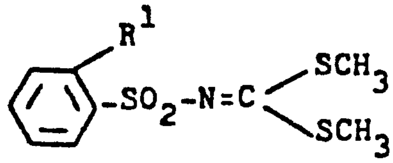

(IV)

,

in der $R^1$ die im Vorstehenden angegebene Bedeutung hat, mit einer Verbindung der allgemeinen Formel

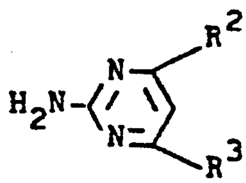

(V)

,

in der $R^2$ und $R^3$ die im Vorstehenden angegebenen Bedeutungen haben.

Verfahren (iii):

Das Verfahren (iii) zur Herstellung der substituierten Phenylsulfonylisothioharnstoff-Derivate der allgemeinen Formel (II) umfaßt die Umsetzung einer Verbindung der allgemeinen Formel

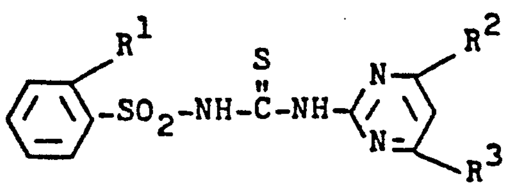

(VI)

,

in der $R^1$, $R^2$ und $R^3$ die im Vorstehenden angegebenen Bedeutungen haben, mit einem Methylierungsmittel in Gegenwart einer Base.

Die vorliegende Erfindung betrifft auch substituierte Phenylsulfonylthioharnstoff-Derivate der allgemeinen Formel (VI), die Vorprodukte des vorstehenden Verfahrens (iii) sind, sowie das folgende Verfahren (iv) zur Herstellung der Derivate der Formel (VI):

Verfahren (iv):

Das Verfahren (iv) zur Herstellung der substituierten Phenylsulfonylthioharnstoff-Derivate der allgemeinen Formel (VI) umfaßt die Umsetzung einer Verbindung der allgemeinen Formel

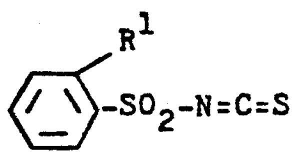 (VII) ,

in der $R^1$ die im Vorstehenden angegebene Bedeutung hat, mit einer Verbindung der oben angegebenen allgemeinen Formel (V).

Die vorliegende Erfindung betrifft auch Unkrautbekämpfungsmittel, die die substituierten Phenylsulfonylguanidin-Derivate der allgemeinen Formel (I) als Wirkstoffe enthalten.

Seitens der Anmelderin wurden ausgedehnte und eingehende Untersuchungen mit dem Ziel durchgeführt, neue Verbindungen mit ausgezeichneter herbizider Wirksamkeit zu gewinnen. Diese Versuche haben nunmehr zur Synthese der substituierten Phenylsulfonylguanidin-Derivate der allgemeinen Formel (I) geführt. Es wurde gefunden, daß diese Verbindungen neue, nicht in der Literatur beschriebene Verbindungen sind und eine herausragende

Wirksamkeit der Unkrautbekämpfung besitzen. Es wurde außerdem gefunden, daß diese Verbindungen bei ihrer Anwendung als Unkrautbekämpfungsmittel für bewässerte Reisfelder eine ausgezeichnete selektive Unkraut- Bekämpfungswirkung gegenüber Reisfeld-Unkräutern zeigen, ohne daß sie gegenüber den Reispflanzen Phytotoxizität ausüben.

Die Verbindungen der allgemeinen Formel (I) gemäß der vorliegenden Erfindung sind völlig neue Verbindungen, die in der Literatur bisher nicht beschrieben sind. Sie sind strukturell durch die Tatsache gekennzeichnet, daß sie auf dem Guanidin-Gerüst aufgebaut sind und eine an das N-Atom in der 1-Stellung gebundene 2-Biphenylsulfonyl-Gruppe oder 2-Phenoxyphenylsulfonyl-Gruppe, eine an das N-Atom in der 2-Stellung gebundene substituierte Pyrimidin-2-yl-Gruppe und, gebunden an das N-Atom in der 3-Stellung, ein Wasserstoff-Atom oder eine niedere Alkyl-Gruppe oder Alkoxy-Gruppe aufweisen. Es wurde gefunden, daß aufgrund dieser einzigartigen chemischen Struktur diese Verbindungen die oben genannten hervorragenden biologischen Kennwerte erlangen können.

Die Verbindungen der allgemeinen Formeln (II), (IV), (VI) und (VII), die Vorprodukte und Zwischenprodukte für die Herstellung der substituierten Phenylsulfonylguanidin-Derivate der allgemeinen Formel (I) sind, sind ebenfalls völlig neue Verbindungen, die in der Literatur bisher nicht beschrieben sind. Von diesen Zwischenprodukten besitzen die substituierten Phenylsulfonylisothioharnstoff-Derivate der allgemeinen Formel (II) und die substituierten Phenylsulfonylthioharnstoff-Derivate der allgemeinen Formel (VI) selbst ausgezeichnete Wirksamkeit der Unkrautbekämpfung und sind nicht

nur als Zwischenprodukte für die Herstellung anderer wertvoller Verbindungen, sondern auch als Endprodukte und als Unkrautbekämpfungsmittel von Nutzen.

Aus diesem Grunde ist es das Ziel der vorliegenden Erfindung, substituierte Phenylsulfonylguanidin-Derivate der allgemeinen Formel (I), Vorprodukte derselben, Verfahren zu ihrer Herstellung sowie ihre Verwendung als Unkrautbekämpfungsmittel verfügbar zu machen, insbesondere als selektive Unkrautbekämpfungsmittel gegen Unkräuter in bewässerten Reisfeldern.

Diese und andere Ziele und Vorteile der vorliegenden Erfindung werden aus der folgenden Beschreibung im einzelnen deutlich.

Die Verbindungen der vorliegenden Erfindung zeigen eine herausragende selektive Bekämpfungswirkung,wenn sie als Mittel zur Bodenbehandlung vor dem Auflaufen oder zur Stengel/Laub/Boden-Behandlung gegen Unkräuter in bewässerten Reisfeldern eingesetzt werden.

Die Verbindungen gemäß der vorliegenden Erfindung weisen eine hohe Sicherheit, eine herausragende Unkrautbekämpfungs-Aktivität und ein breites herbizides Spektrum auf. Beispielsweise sind sie gekennzeichnet durch Unkrautbekämpfungs-Wirksamkeit gegen die folgenden im Wasser wachsenden Reisfeld-Unkräuter ohne jegliche Phytotoxizität gegenüber Reis.

| Pflanzenname: | Lateinische wissenschaftliche Bezeichnung: |
|---|---|
| **Dikotyledonen** | |
| Kikashigusa | Rotala indica Koehne |
| Falsche Pimpernelle (Büchsenkraut) | Lindernia procumbens Philcox |
| Falscher Weiderich (Heusenkraut) | Ludwigia prostrata Roxburgh |
| Breitblättriges Laichkraut | Potamogeton distinctus A. Bennett |
| Amerikanische Wasserwurz (Kreuztännel) | Elatine triandra Schkuhr |
| **Monokotyledonen** | |
| Hühnerhirse | Echinochloa crus-galli Beauvois var. oryzicola Ohwi |
| Monochoria | Monochoria vaginalis Presl |
| Nadel-Sumpfbinse | Eleocharis acicularis Roem. et Schult. |
| Wassernuß | Eleocharis kuroguwai Ohwi |
| Schirmkraut (Cypergras) | Cyperus difformis L. |
| Mizugayatsuri (Cypergras) | Cyperus serotinus Rottboell |
| Urikawa (Pfeilkraut) | Sagittaria pygmaea Miquel, |
| Schmalblättriger Wasserwegerich (Froschlöffel) | Alisma canaliculatum A. Braun et Bouché |
| Simse | Scirpus juncoides Roxburgh var. |

Sie sind weiterhin gekennzeichnet durch Unkrautbekämpfungs-Wirksamkeit gegen die folgenden, auf höher gelegenen Anbauflächen wachsenden Unkräuter.

| Pflanzenname: | Lateinische wissenschaftliche Bezeichnung: |
|---|---|
| Dikotyledonen | |
| Knöterich | Polygonum sp. |
| Gänsefuß | Chenopodium album L. var. centrorubrum Makino |
| Vogelmiere | Stellaria media Villars |
| Kohl-Portulak | Portulaca oleracea Linnaeus |
| Monokotyledonen | |
| Hühnerhirse | Echinochloa crus-galli P.Beauv. |
| Fingergras | Digitaria adscendens Henr. |
| Riedgras | Cyperus microiria Steud. |

Sie sind weiterhin dadurch gekennzeichnet, daß sie keine Phytotoxizität gegen solche Nutzpflanzen auf höher gelegenen Anbauflächen wie Senf, Baumwolle, Möhren, Bohnen, Kartoffeln und Kohlarten (Dikotyledonen) und Mais, Reis, Hafer, Gerste, Weizen sowie Panicum milliaceum und Saccharum officinarum (Monokotyledonen) entfalten.

Es ist ausdrücklich anzumerken, daß die im Vorstehenden angegebenen Pflanzennamen typische Beispiele für die jeweils mit der lateinischen wissenschaftlichen Bezeichnung genannte Gattung sind.

Die Einsetzbarkeit der aktiven Verbindungen gemäß der vorliegenden Erfindung ist nicht auf Unkräuter in bewässerten Reisfeldern und auf höher gelegenen landwirtschaftlichen Anbauflächen beschränkt. Sie sind auch wirksam gegen Unkräuter, die die Weichbinsen (Mattenbinsen; Juncus effusus Linnaeus var. decipiens Buchanan) schädigen, Unkräuter, die auf zeitweise brach liegenden Ländereien auftreten etc.. Die hierin verwendete Bezeichnung "Unkräuter" bezeichnet im weitesten Sinne alle Pflanzen, die an Stellen auftreten, wo sie unerwünscht sind.

Die durch die Formel (I) bezeichneten substituierten Phenylsulfonylguanidin-Derivate gemäß der vorliegenden Erfindung können beispielsweise mittels des folgenden Verfahrens (i) synthetisiert werden.

Verfahren (i)

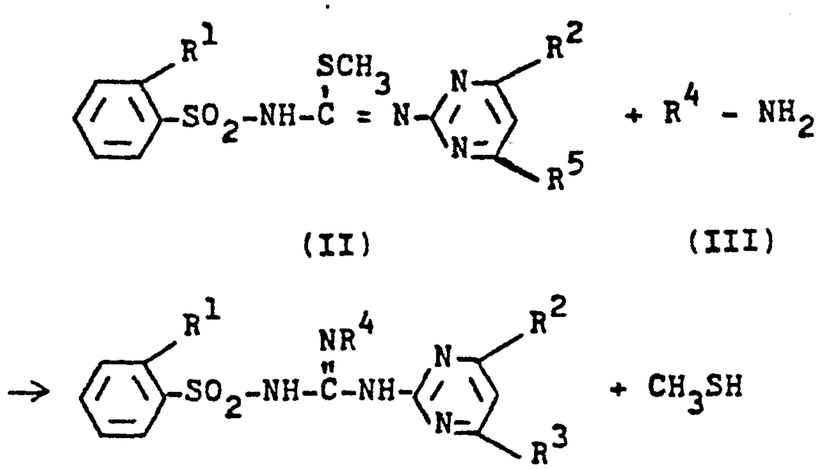

(II) (III)

(I)

In den Formeln haben $R^1$, $R^2$, $R^3$ und $R^4$ die im Vorstehenden angegebenen Bedeutungen. In dem vorstehenden Reaktionsschema bezeichnen

$R^1$ eine Phenyl- oder Phenoxy-Gruppe,

$R^2$ und $R^3$ jeweils eine niedere Alkyl-Gruppe oder eine niedere Alkoxy-Gruppe, speziell solche niederen Alkyl-Gruppen wie Methyl, Ethyl, Propyl, Isopropyl und n- (iso-, sec- oder tert-)Butyl und niedere Alkoxy-Gruppen mit den gleichen niederen Alkyl-Gruppen wie vorstehend,

Nit 163

$R^4$ ein Wasserstoff-Atom, eine niedere Alkyl-Gruppe oder eine niedere Alkoxy-Gruppe, wobei spezielle Beispiele für die niederen Alkyl- und niederen Alkoxy-Gruppen die gleichen sind, wie sie oben für $R^2$ und $R^3$ angegeben sind.

Bei dem durch das vorstehende Reaktionsschema dargestellten Verfahren zur Herstellung der substituierten Phenylsulfonylguanidin-Derivate der allgemeinen Formel (I) zählen zu speziellen Beispielen für die Ausgangs-Verbindung der allgemeinen Formel (II):

1-(2-Biphenylylsulfonyl)-3-(4-methoxy-6-methyl-pyrimidin-2-yl)-2-methylisothioharnstoff;

1-(2-Biphenylylsulfonyl)-3-(4,6-dimethyl-pyrimidin-2-yl)-2-methylisothioharnstoff und

3-(4,6-Dimethyl-pyrimidin-2-yl)-2-methyl-1-(2-phenoxyphenylsulfonyl)isothioharnstoff;

In gleicher Weise sind spezielle Beispiele für die Ausgangs-Verbindung der allgemeinen Formel (III) Ammoniak, O-Methylhydroxylamin, Methylamin und O-Propylhydroxylamin.

Das vorstehende Verfahren wird durch das folgende typische Beispiel im einzelnen erläutert:

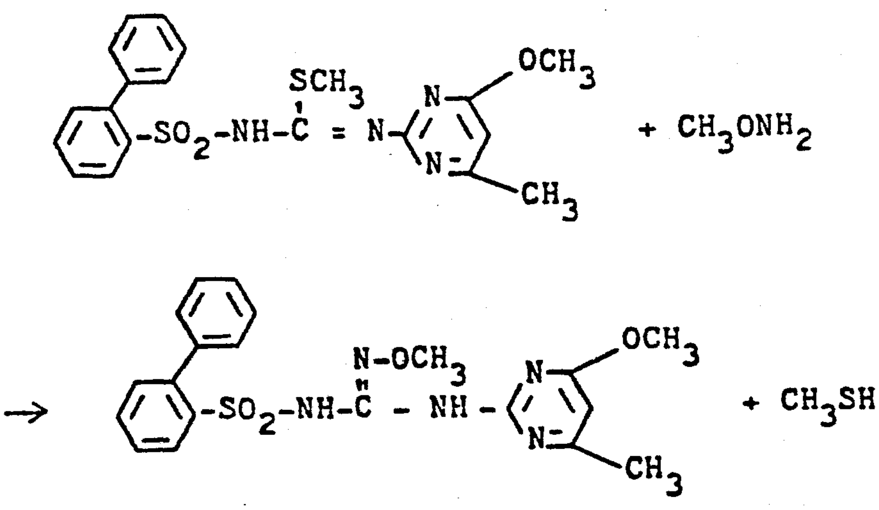

Das vorstehende Verfahren zur Herstellung der Verbindungen gemäß der vorliegenden Erfindung kann zweckmäßigerweise in einem Lösungsmittel oder Verdünnungsmittel durchgeführt werden. Zu diesem Zweck können sämtliche inerten Lösungsmittel oder Verdünnungsmittel verwendet werden.

Beispiele für solche Lösungsmittel oder Verdünnungsmittel umfassen aliphatische, alicyclische und aromatische Kohlenwasserstoffe (die gegebenenfalls chloriert sein können), wie Hexan, Cyclohexan, Petrolether, Ligroin, Benzol, Toluol, Xylol, Methylenchlorid, Chloroform, Kohlenstofftetrachlorid, Ethylenchlorid, Trichloroethylen und Chlorbenzol, Ether wie Diethylether, Methylethylether, Di-isopropylether, Dibutylether, Propylenoxid, Dioxan und Tetrahydrofuran, Ketone wie Aceton, Methylethylketon, Methylisopropylketon und Methylisobutylketon, Nitrile wie Acetonitril, Propionitril und Acrylnitril, Ester wie Ethylacetat und Amylacetat, Säureamide wie Dimethylformamid und Dimethylacetamid, Sulfone und Sulfoxide wie Dimethylsulfoxid, Sulfolan und organische Basen wie Pyridin.

Das obige Verfahren gemäß der vorliegenden Erfindung kann innerhalb eines breiten Temperaturbereichs durchgeführt werden. Im allgemeinen wird die Reaktion bei einer Temperatur zwischen -20°C und dem Siedepunkt der Mischung, zweckmäßigerweise bei einer Temperatur zwischen etwa 0°C und etwa 100°C, durchgeführt. Zweckmäßigerweise wird die Reaktion unter Atmosphärendruck durchgeführt, wenngleich es auch möglich ist, bei erhöhtem oder vermindertem Druck zu arbeiten.

Das substituierte Phenylsulfonylisothioharnstoff-Derivat der allgemeinen Formel (II), das ein Zwischenprodukt ist, kann beispielsweise mittels der folgenden Verfahren (ii) oder (iii) hergestellt werden.

Verfahren (ii)

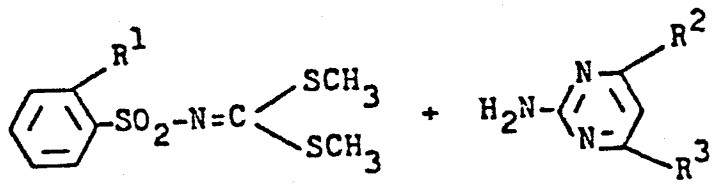

(IV) (V)

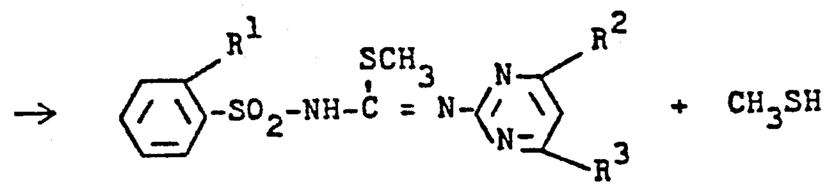

(II)

In den Formeln haben $R^1$, $R^2$ und $R^3$ die im Vorstehenden angegebenen Bedeutungen.

In dem Verfahren zur Herstellung des substituierten Phenylsulfonylisothioharnstoff-Derivats der allgemeinen Formel (II), das durch das vorstehende Reaktionsschema dargestellt ist, sind spezielle Beispiele für die Ausgangs-Verbindung der allgemeinen Formel (IV) Dimethyl-N-(2-biphenylylsulfonyl)carbonimidodithioat und Dimethyl-N-(2-phenoxyphenylsulfonyl)carbonimidodithioat.

In gleicher Weise sind spezielle Beispiele für die Ausgangs-Verbindung der allgemeinen Formel (V) 2-Amino-4,6-dimethylpyrimidin und 2-Amino-4-methoxy-6-methylpyrimidin.

Das vorstehende Verfahren wird durch das folgende typische Beispiel im einzelnen erläutert:

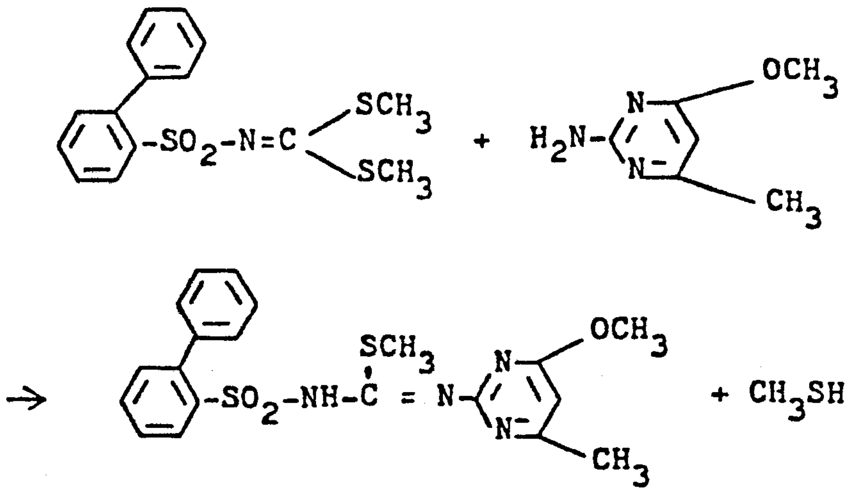

Nach Durchführung des obigen Verfahrens, vorzugsweise unter Verwendung des gleichen inerten Lösungsmittels oder Verdünnungsmittels wie im Vorstehenden, kann das angestrebte Produkt in hoher Reinheit mit hoher Ausbeute gewonnen werden.

Das vorstehende Verfahren kann in wirksamer Weise auch in Gegenwart von, beispielsweise, Natriumhydrid oder Kaliumhydrid durchgeführt werden.

Die vorstehende Reaktion kann innerhalb eines breiten Temperaturbereichs durchgeführt werden, beispielsweise bei einer Temperatur zwischen etwa -20°C und dem Siedepunkt der Mischung, vorzugsweise zwischen etwa 0°C und etwa 100°C. Vorzugsweise wird die Reaktion unter Atmosphärendruck durchgeführt, jedoch ist es auch möglich, bei erhöhtem oder vermindertem Druck zu arbeiten.

Verfahren (iii)

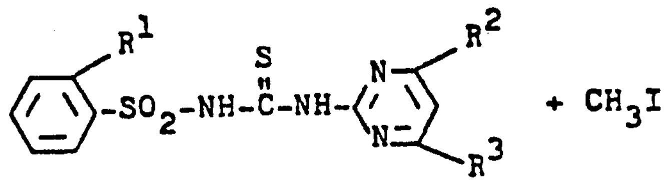


(VI)

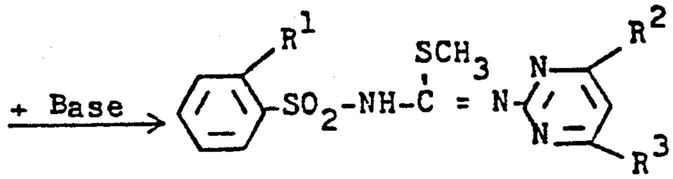


(II)

In den Formeln haben $R^1$, $R^2$ und $R^3$ die im Vorstehenden angegebenen Bedeutungen.

In dem Verfahren zur Herstellung des substituierten Phenylsulfonylisothioharnstoff-Derivats der allgemeinen Formel (II), das durch das vorstehende Reaktionsschema dargestellt ist, umfassen spezielle Beispiele für die Ausgangs-Verbindung der allgemeinen Formel (VI):

1-(2-Biphenylylsulfonyl)-3-(4-methoxy-6-methyl-pyrimidin-2-yl)thioharnstoff;

1-(2-Biphenylylsulfonyl)-3-(4,6-dimethyl-pyrimidin-2-yl)thioharnstoff und

3-(4,6-Dimethyl-pyrimidin-2-yl)-1-(2-phenoxyphenylsulfonyl)thioharnstoff.

Beispiele für das Methylierungsmittel sind Methyliodid, Dimethylsulfat, Methylbromid und Methylchlorid.

Das vorstehende Verfahren sollte in Gegenwart einer Base durchgeführt werden. Beispiele für die Base sind metallisches Natrium, metallisches Kalium, metallisches Lithium, Natriumhydroxid, Kaliumhydroxid und Lithiumhydroxid.

Das vorstehende Verfahren wird durch das folgende typische Beispiel im einzelnen erläutert:

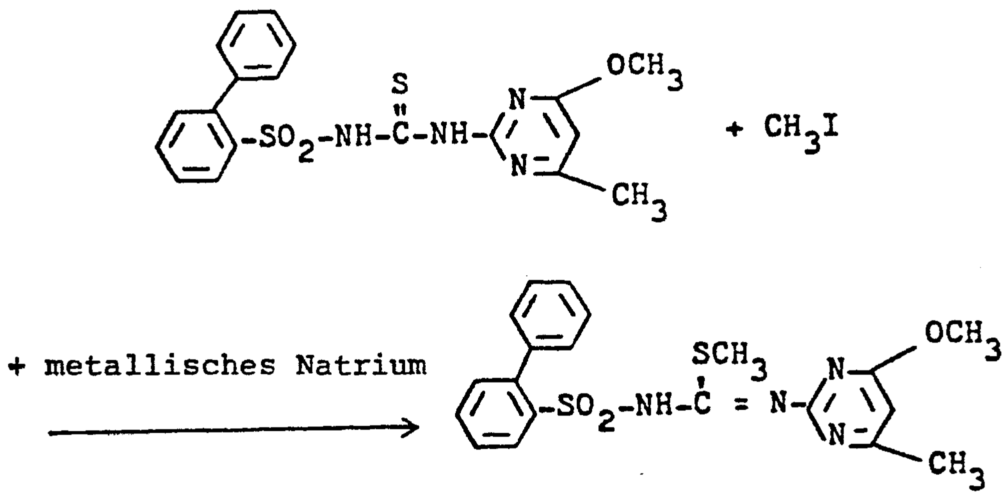


Das obige Verfahren wird vorzugsweise unter Verwendung des gleichen inerten Lösungsmittels oder Verdünnungsmittels wie im Vorstehenden beschrieben durchgeführt, wodurch das angestrebte Produkt in hoher Reinheit mit hoher Ausbeute erhalten wird.

Die vorstehende Reaktion kann innerhalb eines breiten Temperaturbereichs durchgeführt werden, beispielsweise bei einer Temperatur zwischen etwa -20°C und dem Siedepunkt der Mischung, vorzugsweise zwischen etwa 0°C und etwa 100°C. Vorzugsweise wird die Reaktion unter Atmosphärendruck durchgeführt, jedoch ist es auch möglich, bei erhöhtem oder vermindertem Druck zu arbeiten.

Die substituierten Phenylsulfonylthioharnstoff-Derivate der allgemeinen Formel (VI), die Zwischenprodukte sind, können beispielsweise mittels des folgenden Verfahrens (iv) hergestellt werden.

<u>Verfahren (iv)</u>

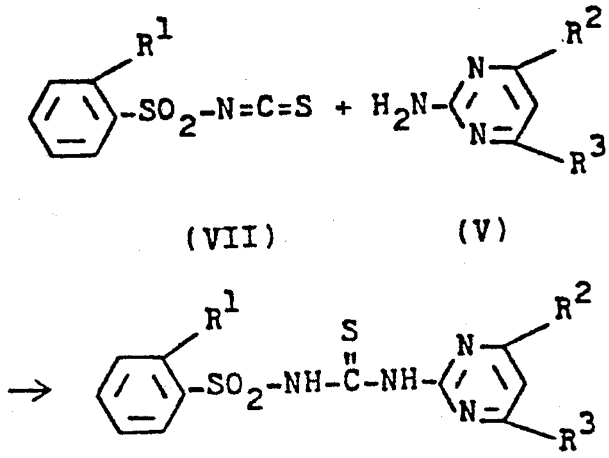

(VII) (V)

(VI)

In den Formeln haben $R^1$, $R^2$ und $R^3$ die im Vorstehenden angegebenen Bedeutungen.

In dem Verfahren zur Herstellung der substituierten Phenylsulfonylthioharnstoff-Derivate der allgemeinen Formel (VI), das durch das vorstehende Reaktionsschema dargestellt ist, sind spezielle Beispiele für die Ausgangs-Verbindung der allgemeinen Formel (VII) 2-Phenylbenzolsulfonylisothiocyanat und 2-Phenoxybenzolsulfonylisothiocyanat. Spezielle Beispiele für die Ausgangs-Verbindung der allgemeinen Formel (V) können die gleichen Verbindungen sein, die für das Verfahren (ii) angegeben wurden.

Das vorstehende Verfahren wird durch das folgende typische Beispiel im einzelnen erläutert:

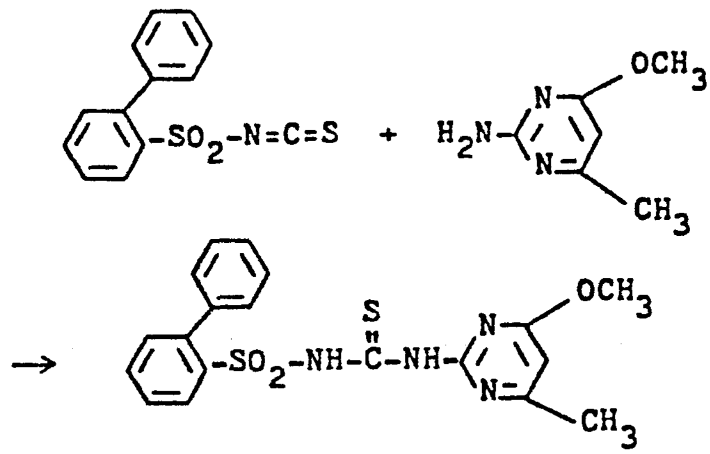

Das obige Verfahren wird vorzugsweise unter Verwendung des gleichen inerten Lösungsmittels oder Verdünnungsmittels wie im Vorstehenden beschrieben durchgeführt, wodurch das angestrebte Produkt in hoher Reinheit mit hoher Ausbeute erhalten wird.

Die vorstehende Reaktion kann innerhalb eines breiten Temperaturbereichs durchgeführt werden, beispielsweise bei einer Temperatur zwischen etwa -20°C und dem Siedepunkt der Mischung, vorzugsweise zwischen etwa 0°C und etwa 100°C. Vorzugsweise wird die Reaktion unter Atmosphärendruck durchgeführt, jedoch ist es auch möglich, bei vermindertem oder erhöhtem Druck zu arbeiten.

Die Reaktion kann durch Verwendung einer organischen Base wie Triethylamin und Diazabicyclooctan als Katalysator gefördert werden.

Zur Verwendung als Herbizide können die durch die allgemeine Formel (I) bezeichneten Verbindungen gemäß der vorliegenden Erfindung als solche unmittelbar nach dem Verdünnen mit Wasser oder in Form verschiedenartiger Präparate, wie sie gewöhnlich unter Verwendung agrochemischer Hilfsstoffe hergestellt werden, mittels der in der Landwirtschaft üblichen Verfahren angewandt werden. Beim praktischen Einsatz werden die herbiziden Zusammensetzungen in ihren verschiedenen Formen entweder unmittelbar oder nach dem Verdünnen mit Wasser auf die gewünschten Konzentrationen aufgebracht. Beispiele für die agrochemisch unbedenklichen Hilfsstoffe, auf die hier Bezug genommen wird, sind Verdünnungsmittel (Lösungsmittel, Streckmittel, Träger), oberflächenaktive Stoffe (Lösungsvermittler, Emulgatoren, Dispergiermittel, Netzmittel), Stabilisatoren, Haftmittel, Aerosol-Treibmittel und Synergisten.

Beispiele für die Lösungsmittel sind Wasser und organische Lösungsmittel, beispielsweise Kohlenwasserstoffe /¯z.B. n-Hexan, Petrolether, Naphtha, Erdöl-Fraktionen (z.B. Paraffinwachse, Kerosin, Leichtöle, Mittelöle, Schweröle), Benzol, Toluol und Xylole_7, halogenierte Kohlenwasserstoffe (z.B. Methylenchlorid, Kohlenstofftetrachlorid, Trichloroethylen, Ethylenchlorid, Ethylendichlorid, Chlorbenzol und Chloroform), Alkohole (z.B. Methylalkohol, Ethylalkohol, Propylalkohol und Ethylenglycol), Ether (z.B. Ethylether, Ethylenoxid und Dioxan), Alkohol-ether (z.B. Ethylenglycol-monomethylether), Ketone (z.B. Aceton und Isophoron), Ester (z.B. Ethylacetat und Amylacetat), Amide (z.B. Dimethylformamid und Dimethylacetamid) und Sulfoxide (z.B. Dimethylsulfoxid).

Beispiele für die Streckmittel oder Träger umfassen anorganische Pulver, beispielsweise Schwefel, Löschkalk, Magnesiumkalk, Gips, Calciumcarbonat, Siliciumdioxid, Perlit, Bimsstein, Calcit, Diatomeenerde, amorphes Siliciumdioxid, Aluminiumoxid, Zeolithe und Tonminerale (z.B. Pyrophyllit, Talkum, Montmorillonit, Beidellit, Vermikulit, Kaolinit und Glimmer), pflanzliche Pulver wie beispielsweise Getreidepulver, Stärkearten, verarbeitete Stärkearten, Zucker, Glucose und zerkleinerte Stengel von Pflanzen, sowie Pulver aus synthetischen Harzen wie Phenol-Harzen, Harnstoff-Harzen und Vinylchlorid-Harzen.

Beispiele für die oberflächenaktiven Mittel umfassen anionische oberflächenaktive Mittel wie Alkylschwefelsäureester (z.B. Natriumlaurylsulfat), Arylsulfonsäure-Salze (z.B. Alkylarylsulfonsäure-Salze und Natriumalkylnaphthalinsulfonate), Bernsteinsäure-Salze und Salze von Schwefelsäureestern von Polyethylenglycol-alkylarylethern, kationische oberflächenaktive Mittel wie Alkylamine (z.B. Laurylamin, Stearyltrimethylammoniumchlorid und Alkyldimethylbenzylammoniumchlorid) und Polyoxyethylenalkylamine, nicht-ionische oberflächenaktive Mittel wie Polyoxyethylenglycolether (z.B. Polyoxyethylenalkylarylether und deren Kondensationsprodukte), Polyoxyethylenglycolester (z.B. Polyoxyethylenfettsäureester) und Ester mehrwertiger Alkohole (z.B. Polyoxyethylensorbitan-monolaurat) sowie amphotere oberflächenaktive Mittel.

Beispiele für andere Hilfsstoffe umfassen Stabilisatoren, Haftmittel (z.B. landwirtschaftliche Seifen, Casein-Kalk, Natriumalginat, Polyvinylalkohol, Haftmittel vom Vinylacetat-Typ und Acryl-Haftmittel), wirkungsverlängernde Mittel, Dispersions-Stabilisatoren (z.B. Casein, Tragant, Carboxymethylcellulose und Polyvinylalkohol) und synergistische Mittel.

Die Verbindungen der vorliegenden Erfindung können mittels der allgemein auf dem Gebiet der Agrochemikalien gebräuchlichen Verfahren zu verschiedenen Präparaten formuliert werden. Beispiele für solche Anwendungsformen sind emulgierbare Konzentrate, Öle, benetzbare Pulver, lösliche Pulver, Suspensionen, Stäubemittel, Granulat, pulvrige Mittel und Kapseln.

Die Unkrautbekämpfungsmittel gemäß der vorliegenden Erfindung können von etwa 0,001 bis etwa 100 Gew.-%, vorzugsweise etwa von 0,005 bis etwa 95 Gew.-% der vorerwähnten Wirkstoffe enthalten.

Für den praktischen Gebrauch beträgt die geeignete Menge der aktiven Verbindung in den vorgenannten Mitteln in ihren verschiedenen Formen und gebrauchsfertigen Präparaten beispielsweise etwa 0,01 bis etwa 95 Gew.-%, vorzugsweise etwa 0,05 bis etwa 60 Gew.-%.

Der Gehalt des Wirkstoffs kann in geeigneter Weise je nach der Form des Präparats, dem Verfahren, Zweck, der Zeit und dem Ort seiner Anwendung, dem Zustand des Auftretens der Unkräuter etc. variiert werden.

Erforderlichenfalls können die Verbindungen gemäß der vorliegenden Erfindung weiterhin auch in Kombination mit anderen Agrochemikalien verwendet werden, beispielsweise mit Insektiziden, Fungiziden, Mitiziden,

Nematiziden, Anti-Virus-Mitteln, Herbiziden, Pflanzen-Wachstumsregulatoren und Lockstoffen /¯z.B. Organophosphorester-Verbindungen, Carbamat-Verbindungen, Dithio-(oder thiol)carbamat-Verbindungen, organischen Chlor-Verbindungen, Dinitro-Verbindungen, organischen Schwefel- oder Metall-Verbindungen, Antibiotika, substituierten Diphenylether-Verbindungen, Harnstoff-Verbindungen und Triazin-Verbindungen_7 und/oder Düngemitteln.

Die den im Vorstehenden bezeichneten Wirkstoff enthaltenden verschiedenartigen Mittel und gebrauchsfertigen Präparate können mittels verschiedener Verfahren zur Anwendung gebracht werden, wie sie allgemein für das Aufbringen von Agrochemikalien gebräuchlich sind, beispielsweise durch Sprühen (Versprühen von Flüssigkeiten, Vernebeln, Zerstäuben, Stäuben, Streuen von Granulat, Wasser-Oberflächenbehandlung und Gießen) und Bodenbehandlung (Vermischen mit dem Boden und Streuen). Sie können auch mittels des sogenannten Ultra-Low-Volume-Sprühverfahrens aufgebracht werden. Nach diesem Verfahren kann der Wirkstoff sogar in einer Konzentration von 100 % zur Anwendung gelangen.

Die Aufwandmenge pro Flächeneinheit beträgt beispielsweise etwa 0,1 bis etwa 3,0 kg/ha, vorzugsweise etwa 0,2 bis etwa 1 kg/ha. In besonders gelagerten Fällen können oder sollten sogar die Aufwandmengen außerhalb des angegebenen Bereichs liegen.

Gemäß der vorliegenden Erfindung wird ein Unkrautbekämpfungsmittel zur Verfügung gestellt, das als Wirkstoff eine Verbindung der allgemeinen Formel (I) sowie ein Verdünnungsmittel (ein Lösungsmittel und/oder ein

Streckmittel und/oder einen Träger) und/oder ein oberflächenaktives Mittel und, falls weiterhin erforderlich, einen Stabilisator, ein Haftmittel, ein synergistisches Mittel etc. enthält.

Die vorliegende Erfindung macht weiterhin ein Verfahren zur Unkrautbekämpfung verfügbar, das darin besteht, daß eine Verbindung der allgemeinen Formel (I) allein oder in Form einer Mischung mit einem Verdünnungsmittel (einem Lösungsmittel und/oder einem Streckmittel und/oder einem Träger) und/oder einem oberflächenaktiven Mittel und, falls weiterhin erforderlich, einem Stabilisator, einem Haftmittel, einem synergistischen Mittel etc. auf Unkräuter und/oder ihren Lebensraum aufgebracht wird.

Die vorliegende Erfindung wird durch die folgenden Beispiele näher erläutert, ist jedoch nicht auf diese beschränkt.

Beispiel 1

Synthese eines Zwischenprodukts der allgemeinen Formel (II):

12,3 g 2-Amino-4,6-dimethylpyrimidin wurden in 300 ml Dimethylformamid suspendiert, und 4 g Natriumhydroxid (ölig, 60 %) wurden zugesetzt. Die Mischung wurde 1 h bei Raumtemperatur gerührt. Weiterhin wurden 33,7 g Dimethyl-N-(2-biphenylylsulfonyl)carbonimidodithioat hinzugefügt, und die Mischung wurde einen Tag und eine Nacht bei Raumtemperatur gerührt. Die Reaktionsmischung wurde in 2 l Wasser gegossen und filtriert. Das Filtrat

wurde schwach angesäuert, wonach weiße Kristalle ausfielen. Die Kristalle wurden durch Filtration gesammelt und aus Ethanol umkristallisiert, wonach 29,2 g des angestrebten 1-(2-Biphenylylsulfonyl)-3-(4,6-dimethylpyrimidin-2-yl)-2-methylisothioharnstoffs der nachstehenden Formel erhalten wurden. Schmp. 154-157°C.

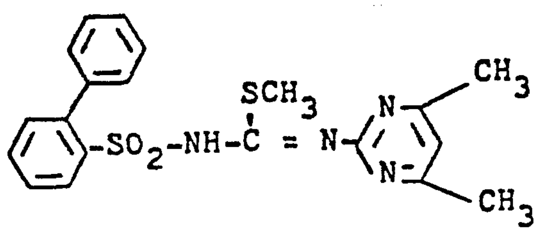

(Verbindung Nr. II-1),

Nach im wesentlichen der gleichen Arbeitsweise wie im vorstehenden Beispiel 1 wurden die folgenden Verbindungen synthetisiert.

| | |
|---|---|
| (Verbindung Nr. II-2) | 1-(2-Biphenylylsulfonyl)-3-(4-methoxy-6-methyl-pyrimidin-2-yl)-2-methylisothioharnstoff, Schmp. 138-140°C; |
| (Verbindung Nr. II-3) | 3-(4,6-Dimethylpyrimidin-2-yl)-2-methyl-1-(2-phenoxyphenylsulfonyl)isothioharnstoff, Schmp. 143-145°C. |

Ein Literaturbeispiel für die Synthese des in Beispiel 1 verwendeten Dimethyl-N-(2-biphenylylsulfonyl)carbonimidodithioats ist nachstehend angegeben.

Literatur-Synthesebeispiel 1

116,5 g 2-Phenylbenzolsulfonamid wurden in 500 ml Dimethylformamid gelöst, und 38 g Carbondisulfid und 28 g

Nit 163

Kaliumhydroxid wurden zu der entstandenen Lösung hinzugefügt. Die Mischung wurde 7 h bei Raumtemperatur gerührt. Der Reaktionsmischung wurden 28 g Kaliumhydroxid zugesetzt, und die Mischung wurde bei Raumtemperatur 2 h gerührt. Dann wurden 400 ml Ethylacetat tropfenweise hinzugegeben, wodurch blaßgelbe Kristalle ausfielen. Die Kristalle wurden filtriert und mit Ethylacetat gewaschen. Das entstandene Kalium-N-(2-biphenylylsulfonyl)carbonimidodithioat wurde in 400 ml Dimethylformamid gelöst, und 126 g Dimethylsulfat wurden tropfenweise im Laufe von 1 h bei 60°C zu der Lösung hinzugegeben. Nach der Zugabe wurde die Mischung 2 h auf 80°C erhitzt. Nach dem Abkühlen wurde die Reaktionsmischung in 1 l Wasser gegossen, und die ausgeschiedenen Kristalle wurden durch Filtration gesammelt. Danach wurden 270 g des gesuchten Dimethyl-N-(2-biphenylylsulfonyl)-carbonimidodithioat der nachstehenden Formel erhalten; Schmp.: 115-117°C.

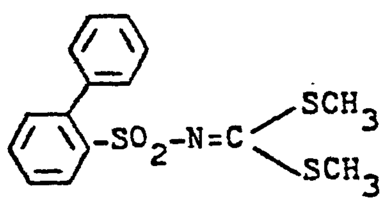

(Verbindung Nr. IV-1)

Nach im wesentlichen der gleichen Arbeitsweise wie im vorstehenden Literatur-Synthesebeispiel 1 wurde Dimethyl-N-(2-phenoxyphenylsulfonyl)carbonimidodithioat (Verbindung Nr. IV-2) synthetisiert.

Beispiel 2

Synthese eines Zwischenprodukts der allgemeinen Formel (II):

3,98 g 1-(2-Biphenylylsulfonyl)-3-(4,6-dimethylpyrimidin-2-yl)thioharnstoff wurden in 50 ml trockenem Acetonitril suspendiert, und eine Lösung von 0,3 g metallischem Natrium in 10 ml Ethanol wurde hinzugefügt, und die Mischung wurde 30 min unter Rückfluß erhitzt. Nach dem Abkühlen wurden 1,85 g Methyliodid hinzugefügt, und die Mischung wurde 5 h unter Rückfluß erhitzt. Nach dem Abkühlen wurde die Reaktionsmischung in 100 ml Wasser gegossen. Das Produkt wurde mit Methylenchlorid extrahiert. Das Methylenchlorid wurde unter vermindertem Druck abgedampft, und der Rückstand wurde aus Ethanol umkristallisiert, wonach 1,6 g 1-(2-Biphenylylsulfonyl)-3-(4,6-dimethyl-pyrimidin-2-yl)-2-methylisothioharnstoff (Verbindung II-1), das gleiche Produkt wie in Beispiel 1, erhalten wurden; Schmp.: 154-157°C.

Ein Beispiel für die Synthese des in Beispiel 2 verwendeten 1-(2-Biphenylylsulfonyl)-3-(4,6-dimethylpyrimidin-2-yl)thioharnstoffs ist im Folgenden dargestellt.

Beispiel 3

Synthese eines Zwischenprodukts der allgemeinen Formel (VI):

77 g Kalium-N-(2-biphenylylsulfonyl)carbonimidodithioat, das gemäß Literatur-Synthesebeispiel 1 hergestellt wurde, wurden in 100 ml Toluol suspendiert, und 37 ml Thionylchlorid wurden zu der Suspension im Laufe von 1 h bei 0°C bis 10°C hinzugefügt. Nach der Zugabe wurde

die Mischung 2 h bei Raumtemperatur gerührt. Die Reaktionsmischung wurde filtriert, und das Filtrat wurde unter vermindertem Druck konzentriert. Die Destillation unter vermindertem Druck lieferte 2-Phenylbenzolsulfonylisothiocyanat. Andererseits wurden 12,3 g 2-Amino-4,6-dimethyl-pyrimidin in 300 ml trockenem Toluol suspendiert, und 27,5 g 2-Phenylbenzolsulfonylisothiocyanat, das wie vorstehend hergestellt wurde, wurden hinzugegeben. Die Mischung wurde 8 h unter vermindertem Druck zum Rückfluß erhitzt. Nach dem Abkühlen wurde der entstandene Niederschlag durch Filtration gesammelt und aus Ethanol umkristallisiert, wonach 31 g des gesuchten 1-(2-Biphenylylsulfonyl)-3-(4,6-dimethylpyrimidin-2-yl)thioharnstoffs der nachstehenden Formel erhalten wurden; Schmp.: 203-204°C.

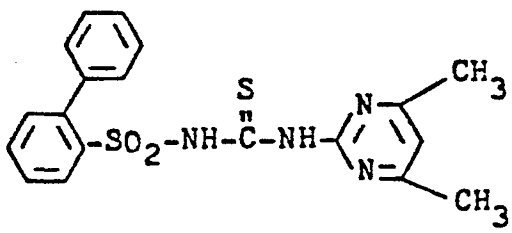

(Verbindung Nr. VI-1)

Nach im wesentlichen der gleichen Arbeitsweise wie im vorstehenden Beispiel 3 wurden die folgenden Verbindungen synthetisiert.

(Verbindung Nr. VI-2) 1-(2-Biphenylylsulfonyl)-3-(4-methoxy-6-methyl-pyrimidin-2-yl)thioharnstoff, Schmp. 203-204°C;

(Verbindung Nr. VI-3) 3-(4,6-Dimethylpyrimidin-2-yl)-1-(2-phenoxyphenylsulfonyl)thioharnstoff.

Beispiel 4

Synthese der Endprodukt-Verbindung der allgemeinen Formel (I):

4,12 g 1-(2-Biphenylylsulfonyl)-3-(4,6-dimethylpyrimidin-2-yl)-2-methylisothioharnstoff wurden in 50 ml Dioxan gelöst, und 2,35 g O-Methylhydroxylamin wurden zu der Lösung hinzugefügt. Die Mischung wurde 5 h unter Rückfluß erhitzt. Nach dem Abkühlen der Reaktionsmischung wurde das Lösungsmittel unter vermindertem Druck abgedampft. Der Rückstand wurde aus Ethanol umkristallisiert, wonach 2,6 g N-(2-Biphenylylsulfonyl)-N'-(4,6-dimethylpyrimidin-2-yl)-N"-(methoxy)guanidin der nachstehenden Formel erhalten wurden; Schmp. 190-192°C.

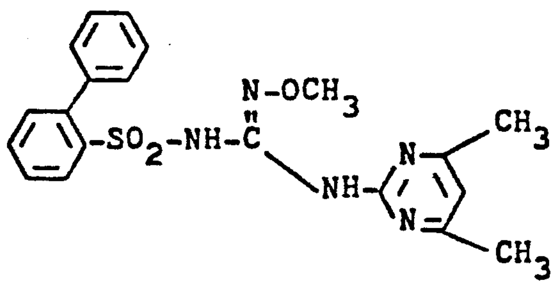

(Verbindung Nr. I-1)

Nach im wesentlichen der gleichen Arbeitsweise wie im vorstehenden Beispiel 4 wurden die folgenden Verbindungen synthetisiert.

(Verbindung Nr. I-2) N-(2-Biphenylylsulfonyl)-N'-(4-methoxy-6-methyl-pyrimidin-2-yl)-N"-(methoxy)guanidin, Schmp. 140-141°C;

(Verbindung Nr. I-3) N-(2-Phenoxyphenylylsulfonyl)-N'-(4,6-dimethyl-pyrimidin-2-yl)-N"-(methoxy)guanidin, Schmp. 139-142°C;

| | |
|---|---|
| (Verbindung Nr. I-4) | N-(2-Biphenylylsulfonyl)-N'-(4-methoxy-6-methyl-pyrimidin-2-yl)-N"-(propoxy)guanidin; |
| (Verbindung Nr. I-5) | N-(2-Biphenylylsulfonyl)-N'-(4-methoxy-6-methyl-pyrimidin-2-yl)-N"-(methyl)guanidin; |
| (Verbindung Nr. I-6) | N-(2-Biphenylylsulfonyl)-N'-(4-methoxy-6-methyl-pyrimidin-2-yl)guanidin. |

Beispiel 5

Benetzbares Pulver.

15 Teile der Verbindung Nr. I-1 der Erfindung, 80 Teile eines Gemisches (1:5) aus pulvriger Diatomeenerde und Tonpulver, 2 Teile Natriumalkylbenzolsulfonat und 3 Teile Natriumalkylnaphthalinsulfonat/Formaldehyd-Kondensat werden pulverisiert und zu einem benetzbaren Pulver vermischt. Dieses wird mit Wasser verdünnt und auf Unkräuter und/oder ihren Lebensraum aufgetropft.

Beispiel 6

Emulgierbares Konzentrat.

30 Teile der Verbindung Nr. I-1 der Erfindung, 55 Teile Xylol, 8 Teile Polyoxyethylen-alkylphenylether und 7 Teile Calciumalkylbenzolsulfonat werden unter Rühren miteinander vermischt, wodurch ein emulgierbares Konzentrat hergestellt wird. Dieses wird mit Wasser verdünnt und auf Unkräuter und/oder ihren Lebensraum aufgetropft.

Beispiel 7

Stäubemittel.

Die Verbindung Nr. I-3 der Erfindung (2 Teile) und 98 Teile Tonpulver werden pulverisiert und gemischt, wodurch ein Stäubemittel hergestellt wird. Dieses wird über Unkräutern und/oder ihrem Lebensraum ausgestreut.

Beispiel 8

Stäubemittel.

Die Verbindung Nr. I-4 der Erfindung (1,5 Teile), 0,5 Teile Isopropylhydrogenphosphat (PAP) und 98 Teile Tonpulver werden pulverisiert und gemischt, wodurch ein Stäubemittel hergestellt wird. Dieses wird über Unkräutern und/oder ihrem Lebensraum ausgestreut.

Beispiel 9

Granulat.

Wasser (25 Teile) wird zu einer Mischung aus 10 Teilen der Verbindung Nr. I-2 der Erfindung, 30 Teilen Bentonit (Montmorillonit), 58 Teilen Talkum und 2 Teilen Ligninsulfonat zugesetzt, und das Gemisch wird gut geknetet. Die Mischung wird mittels eines Extruder-Granulators zu einem Granulat mit einer Korngröße von 0,43 bis 2,0 mm (10 bis 40 mesh) verarbeitet und bei 40°C bis 50°C getrocknet, wodurch ein Granulat gebildet wird. Dieses wird über Unkräutern und/oder ihrem Lebensraum ausgestreut.

Beispiel 10

Granulat.

Ein Drehmischer wird mit 95 Teilen Tonmineral-Teilchen mit einer Teilchengrößen-Verteilung im Bereich von 0,2 bis 2 mm beschickt, und unter Drehen des Mischers werden 5 Teile der in einem organischen Lösungsmittel gelösten Verbindung Nr. I-5 der Erfindung zur gleichmäßigen Benetzung auf die Tonmineral-Teilchen aufgesprüht. Die Teilchen werden dann bei 40°C bis 50°C getrocknet, wodurch ein Granulat gebildet wird. Dieses wird über Unkräutern und/oder ihrem Lebensraum ausgestreut.

Beispiel 11

Prüfung der Wirkung gegen im Wasser wachsende Reisfeld-Unkräuter durch Behandlung des Bodens sowie der Stengel und Blätter unter Bewässerungs-Bedingungen (Topf-Test):

---

Wirkstoff-Präparat:

Träger: 5 Gewichtsteile Aceton;

Emulgator: 1 Gewichtsteil Benzyloxypolyglycolether.

Zur Herstellung eines Wirkstoff-Präparats wird 1 Gew.-Teil jeder der aktiven Verbindungen mit dem Träger und Emulgator in den vorbezeichneten Mengen vermischt, und eine vorher festgelegte Menge des resultierenden emulgierbaren Konzentrats wurde mit Wasser verdünnt.

Test-Verfahren:

Reis-Kulturboden wurde in Wagner-Töpfe (2 dm²) gefüllt, und zwei Reis-Setzlinge (Varietät: Kinnampu) im 2- bis

3-Blattstadium (Pflanzenhöhe etwa 10 cm) wurden in jeden Topf umgepflanzt. Samen von Monochoria vaginalis, Scirpus juncoides und breitblättrigen Unkräutern, kleine Stücke von Eleocharis acicularis L. und Knollen von Cyperus serotinus und Sagittaria pygmaea wurden eingesetzt, und der Boden in den Töpfen wurde in feuchtem Zustand gehalten. Etwa 7 bis 9 Tage nach der Einsaat wurden die Töpfe mit Wasser bis zu einer Tiefe von etwa 6 cm gefüllt. Eine vorher festgelegte Menge des Wirkstoffs wurde in Form einer Emulsion mittels einer Pipette dem Wasser zugegeben. Nach der Behandlung wurden die Töpfe im Laufe von zwei Tagen mit einer Geschwindigkeit von 2 bis 3 cm pro Tag bewässert, während das Wasser aus den Töpfen heraussickern konnte. Danach wurde die Wassertiefe in den Töpfen bei etwa 3 cm gehalten. In der vierten Woche nach der chemischen Behandlung wurden die Unkrautbekämpfungs-Wirkung und der Grad der Phytotoxizität mit Hilfe einer Skala von 0 bis 5 nach dem folgenden Maßstab bewertet.

| Bewertung | Unkrautbekämpfungs-Wirkung (bezogen auf die unbehandelte Fläche) |
|---|---|
| 5 | wenigstens 95 % (verdorrt) |
| 4 | wenigstens 80 %, jedoch weniger als 95 % |
| 3 | wenigstens 50 %, jedoch weniger als 80 % |
| 2 | wenigstens 30 %, jedoch weniger als 50 % |
| 1 | wenigstens 10 %, jedoch weniger als 30 % |
| 0 | weniger als 10 % (unwirksam) |

| Bewertung | Phytotoxizitätsrate gegenüber Reis (bezogen auf die unbehandelte Fläche) |
|---|---|
| 5 | wenigstens 90 % (Ausrottung) |
| 4 | wenigstens 50 %, jedoch weniger als 90 % |
| 3 | wenigstens 30 %, jedoch weniger als 50 % |
| 2 | wenigstens 10 %, jedoch weniger als 30 % |
| 1 | mehr als 0 %, jedoch weniger als 10 % |
| 0 | 0 % (keine Phytotoxizität) |

Die Ergebnisse sind in Tabelle 1 aufgeführt.

Tabelle 1

| Verbindung Nr. | Wirkstoff-Menge kg/ha | Herbizide Wirkung Unkräuter A | B | C | D | E | F | Phytotoxität gegen Reis |
|---|---|---|---|---|---|---|---|---|
| I-1 | 0,5 | 5 | 5 | 5 | 5 | 4 | 5 | 0 |
| I-2 | 0,5 | 5 | 5 | 5 | 5 | 4 | 5 | 0 |
| I-3 | 0,5 | 5 | 5 | 5 | 5 | 4 | 5 | 0 |
| I-4 | 0,5 | 5 | 5 | 5 | 5 | 4 | 5 | 0 |
| I-5 | 0,5 | 5 | 5 | 5 | 5 | 4 | 5 | 0 |
| Vergleich: | | | | | | | | |
| Simetryn | 0,5 | 4 | 3 | 5 | 5 | 2 | 2 | 2 |

Anmerkung:

1. Die Nummern der Verbindungen entsprechen den hierin angegebenen.

2. Die Symbole A bis F bezeichnen die nachstehenden Unkräuter:
   A: Eleocharis acicularis
   B: Scirpus juncoides
   C: Monochoria vaginalis
   D: Breitblättrige Unkräuter (darunter Lindernia procumbens, Rotala indica, Elatine triandra etc.)
   E: Cyperus serotinus
   F: Sagittaria pygmaea
3. Vergleich: Simetryn (common name): 2,4-Bis(ethylamino)-6-methylthio-1,3,5-triazin.

Andere aktive Verbindungen der allgemeinen Formel (I) gemäß der vorliegenden Erfindung als die in Beispiel 11 aufgeführten zeigten den gleichen hervorragenden Wirkungsgrad.

Mittels des gleichen Testverfahrens wie im Beispiel 11 wurden die herbiziden Wirkungen der Verbindungen der allgemeinen Formeln (II) und (VI), die neue Zwischenprodukte gemäß der vorliegenden Erfindung sind, getestet. Die Ergebnisse sind in Tabelle 2 aufgeführt.

Tabelle 2

| Verbindung | Wirkstoff-Menge | Herbizide Wirkung Unkräuter | | | | | | Phytotoxität gegen Reis |
|---|---|---|---|---|---|---|---|---|
| Nr. | kg/ha | A | B | C | D | E | F | |
| II-1 | 0,5 | 5 | 5 | 5 | 5 | 4 | 5 | 0 |
| VI-1 | 0,5 | 5 | 5 | 5 | 5 | 4 | 5 | 0 |

Anmerkung:

1. Die Nummern der Verbindungen entsprechen den im Vorstehenden angegebenen.
2. Die Symbole für die Unkräuter sind die gleichen wie in der Tabelle 1.

Patentansprüche:

1. Substituierte Phenylsulfonylguanidin-Derivate der allgemeinen Formel (I)

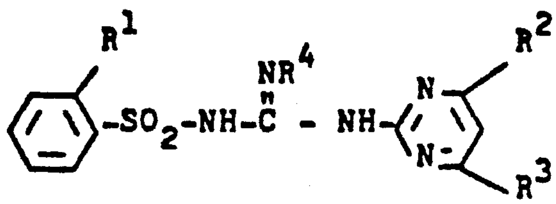

(I)

in der
$R^1$ eine Phenyl- oder Phenoxy-Gruppe bezeichnet,
$R^2$ und $R^3$ jeweils eine niedere Alkylgruppe oder eine niedere Alkoxygruppe bezeichnen und
$R^4$ ein Wasserstoffatom, eine niedere Alkylgruppe oder eine niedere Alkoxygruppe bezeichnet.

2. Verfahren zur Herstellung von substituierten Phenylsulfonylguanidin-Derivaten der allgemeinen Formel (I) gemäß Anspruch 1, dadurch gekennzeichnet, daß man eine Verbindung der allgemeinen Formel (II)

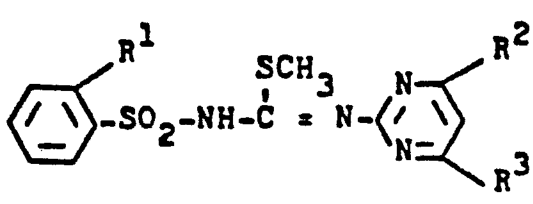

(II)

in der
$R^1$, $R^2$ und $R^3$ die in Anspruch 1 angegebene Bedeutung haben,

mit einer Verbindung der allgemeinen Formel (III)

$$R^4\text{-}NH_2 \qquad (III)$$

in der
$R^4$ die in Anspruch 1 angegebene Bedeutung hat, umsetzt.

3. Herbizide Mittel, gekennzeichnet durch einen Gehalt an mindestens einem substituierten Phenylsulfonylguanidin-Derivat der allgemeinen Formel (I) gemäß Anspruch 1.

4. Verwendung von substituierten Phenylsulfonylguanidin-Derivaten der allgemeinen Formel (I) gemäß Anspruch 1 zur Bekämpfung von unerwünschtem Pflanzenwachstum.

5. Verfahren zur Herstellung von herbiziden Mitteln, dadurch gekennzeichnet, daß man substituierte Phenylsulfonylguanidin-Derivate der allgemeinen Formel (I) gemäß Anspruch 1 mit Streckmitteln und/oder oberflächenaktiven Mitteln vermischt.

6. Substituierte Phenylsulfonylisothioharnstoff-Derivate der allgemeinen Formel (II) gemäß Anspruch 2.

7. Verfahren zur Herstellung von substituierten Phenylsulfonylisothioharnstoff-Derivaten der allgemeinen Formel (II) gemäß Anspruch 2, dadurch gekennzeicnet, daß man eine Verbindung der allgemeinen Formel (IV)

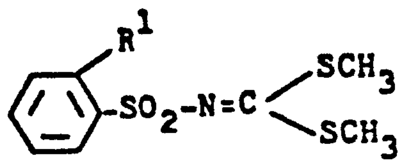
(IV)

in der
$R^1$ die in Anspruch 1 angegebene Bedeutung hat,

mit einer Verbindung der Formel (V)

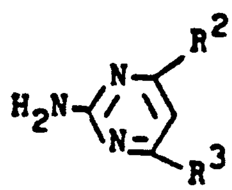
(V)

in der $R^2$ und $R^3$ die in Anspruch 1 angegebenen Bedeutungen haben,
umsetzt.

8. Verfahren zur Herstellung von substituierten Phenylsulfonylisothioharnstoff-Derivaten der allgemeinen Formel (II) gemäß Anspruch 2, dadurch gekennzeichnet, daß man eine Verbindung der allgemeinen Formel (VI)

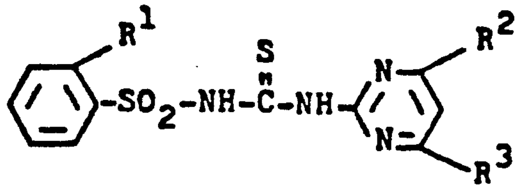

(VI)

in der
$R^1$, $R^3$ und $R^3$ die in Anspruch 1 angegebene Bedeutung haben,
mit einem Methylierungsmittel umsetzt.

9. Substituierte Phenylsulfonylthioharnstoff-Derivate der allgemeinen Formel (VI) gemäß Anspruch 8.

10. Verfahren zur Herstellung von substituierten Phenylsulfonylthioharnstoff-Derivaten der allgemeinen Formel (VI) gemäß Anspruch 8, dadurch gekennzeichnet, daß man eine Verbindung der allgemeinen Formel (VII)

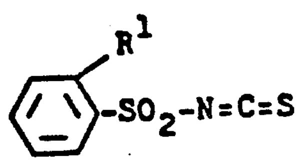

(VII)

in der
$R^1$ die in Anspruch 1 angegebene Bedeutung hat,
mit einer Verbindung der allgemeinen Formel (V) gemäß Anspruch 7 umsetzt.

Europäisches Patentamt

**0136455**

Nummer der Anmeldung

# EUROPÄISCHER RECHERCHENBERICHT

EP 84 10 9024

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| A | EP-A-0 056 969 (NIHON TOKUSHU NOYAKU SEIZO K.K.)<br>* Seiten 12,25-27; Ansprüche *<br>--- | 1,3,10 | C 07 D 239/42<br>C 07 D 239/46<br>A 01 N 47/44 //<br>C 07 C 143/78 |
| A | EP-A-0 074 595 (NIHON TOKUSHU NOYAKU SEIZO K.K.)<br>* Seiten 8,31-34; Ansprüche *<br>--- | 1,3,10 | |
| Y | EP-A-0 005 986 (E.I. DU PONT DE NEMOURS AND COMPANY)<br>* Seiten 7,8,12-16; Ansprüche *<br>--- | 6,7,10 | |
| Y | EP-A-0 043 642 (GULF OIL CORP.)<br>* Seiten 2,3 *<br>--- | 2,8 | |
| Y | EP-A-0 023 422 (E.I. DU PONT DE NEMOURS AND COMPANY)<br>* Seite 38, Beispiel 3 *<br>----- | 9,10 | **RECHERCHIERTE SACHGEBIETE (Int. Cl.4)**<br>C 07 D 239/00<br>A 01 N 47/00 |

Der vorliegende Recherchenbericht wurde für alle Patentansprüche erstellt.

| Recherchenort | Abschlußdatum der Recherche | Prüfer |
|---|---|---|
| DEN HAAG | 08-11-1984 | VAN BIJLEN H. |

**KATEGORIE DER GENANNTEN DOKUMENTEN**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82